# EUROPEAN PATENT SPECIFICATION

(11) **EP 1 924 156 B1**
(45) Date of publication and mention of the grant of the patent: **06.05.2009**
(21) Application number: 06795811.6
(22) Date of filing: 29.08.2006
(51) Int. Cl.: A23L 1/00, A61K 9/50, A23P 1/04

(54) **ENCAPSULATED ACTIVE INGREDIENTS, METHODS OF PREPARATION AND THEIR USE**
EINGEKAPSELTE WIRKSTOFFE, VERFAHREN ZUR VORBEREITUNG UND IHRE VERWENDUNG
INGREDIENTS FONCTIONNELS MULTI-ENCAPSULES, METHODE DE PREPARATION ET LEUR UTILISATION

(30) Priority: 30.08.2005 EP 05107932; 06.09.2005 US 714909 P
(43) Date of publication of application: 28.05.2008
(73) Proprietor: Firmenich S.A., 1211 Geneva 8 (CH)
(72) Inventor: BOUQUERAND, Pierre-Etienne, F-74930 Pers-jussy (FR); DARDELLE, Grégory, F-74520 Jonzier Epagny (FR); GOUIN, Sébastien, CH-1400 Lausanne (CH); SCHLEIFENBAUM, Birgit, CH-1299 Crans-pres-celigny (CH); TROPHARDY, Gil, F-01170 Gex (FR)
(74) Representative: Dale, Gavin Christopher
(86) International application number: PCT/IB2006/052995
(87) International publication number: WO 2007/026307

(56) References cited:
- EP-A- 0 346 879
- WO-A-97/13416
- US-A- 4 894 234
- US-A- 6 039 901
- US-A1- 2002 044 968
- US-A1- 2004 017 017
- US-A1- 2004 037 890
- US-A1- 2005 067 726

## Description

### Technical Field

The present invention relates to particles comprising an active ingredient, a method for preparing the particles, a method for stabilizing an active ingredient encapsulated in coacervated capsules, and a method for masking or preventing undesired or unpleasant taste of an active ingredient intended for oral ingestion.

### Background of the Invention

Encapsulation of active agents, ingredients, molecules or compositions such as nutritional supplements, pharmaceuticals, herbicides, insecticides and many others has the purpose of stabilising a concentrated, easily transportable and optionally processible form of an active ingredient until its release from the encapsulating system at a predetermined place and time. This can vary depending on the nature and purpose of the active ingredient. The prior art discloses various encapsulation technologies adapted to different active ingredients and different places of release and it is clear that many encapsulation systems provide very specific release characteristics tailored to precise environments and times.

The present invention seeks to provide a delivery and/or encapsulation system for encapsulating an active ingredient intended for oral ingestion but to be released not before arrival in the gastro-intestinal tract. The present invention thus deals with active ingredients that are preferably not released in the oral cavity, e.g. because of their undesirable taste and because the active component is more effective if released enterally. Examples of such active ingredients include unpalatable pharmaceuticals, such as bitter tasting active principles intended for oral ingestion, or unpleasant tasting nutritional supplements, such as fish oil, for example, which has a fishy taste and may also have off-notes resulting from oxidation of polyunsaturated fatty acids present in fish oil.

Against this background, it is clearly a problem to add an active ingredient to a food product while preventing its release during chewing and crunching of the food product.

Furthermore, it is desirable to provide an encapsulation or delivery system, which is capable of effectively stabilising an active ingredient when it is exposed to an oxygen-containing environment, e.g. the atmosphere. Thus, it is also an objective to provide an encapsulated ingredient in a form that permits a prolonged shelf life.

It is also desirable to provide high amounts of active ingredient in particles or capsules, while at the same time providing the above-indicated properties (e.g. determined release and stability). In other words, it is particularly desirable to provide an encapsulation system with an increased high load of active ingredient.

Further benefits which are desirable include the possibility of accurate dosing of active ingredient in an encapsulation system/matrix or in a product comprising the encapsulation system, and the ability of accurately dosing particles or capsules in an edible product, in order to provide a specific amount of active ingredient.

WO 97/13416 discloses a double encapsulation process by which flavorant compositions may be encapsulated. The disclosed process provides for micro-encapsulating a flavouring agent by coacervation, and, spray-drying the flavorant composition. Given that flavouring compositions are encapsulated it derives that this reference results in release in the oral cavity. This is in contrast to the present invention, which seeks to prevent release in the oral cavity but aims at release in the gastro-intestinal tract instead.

Lamprecht et al, in "Influences of process parameters on preparation of microparticle used as a carrier system for σ-3 unsaturated fatty acid esters used in supplementary nutrition". J. Microencapsulation, 2001, vol. 18, 3, 347-357, disclose microparticles containing ethyl ester of eicosapentaenoic acid, prepared by coacervation and dried in varying ways. Drying the microcapsules by spray drying was shown to be less effective and give worse protection against oxidation than drying in an ethanol bath. Additionally, no hardening step was performed.

The present invention seeks to address one or more of the abovementioned problems and/or to provide one or more of the abovementioned benefits.

### Summary of the Invention

Remarkably, the present inventors have found a way to provide an effective oxygen barrier to an oxygen-sensitive active ingredient intended for oral administration, and thus to provide an increased stability during shelf life. In addition, when administrated orally and incorporated in a food product, for example, the particles surprisingly release the active ingredient when arriving in the gastrointestinal tract.

Thus according to the present invention, there is provided a particle comprising an active ingredient which is encapsulated in one or more coacervated capsules, the one or more coacervated capsules being further encapsulated in a glassy matrix, wherein the glassy matrix comprises:
(i) 3 - 50wt% of a hydrophobically modified starch, and
(ii) 50 - 97wt% of a starch hydrolysate.

The present invention also provides a method of preparing a particle encapsulating an active ingredient, the method comprising the steps of encapsulating an active ingredient by coacervation to obtain one or more coacervated capsules, and forming a glassy matrix around the one or more coacervated capsules, wherein the glassy matrix comprises:
(i) 3 - 50wt% of a hydrophobically modified starch, and
(ii) 50 - 97wt% of a starch hydrolysate.

In another aspect, the present invention provides the use of a glassy matrix as defined above, the glassy matrix encapsulating one or more coacervated capsules to mask undesired and/or unpleasant taste, off-note or bitterness of an active ingredient intended for oral ingestion.

In yet another aspect, the present invention provides the use of a glassy matrix as defined above, the glassy matrix encapsulating one or more coacervated capsules to stabilise an active ingredient within the capsule.

In still another aspect, the present invention provides a food product comprising a particle according to the invention.

The present invention has a number of unexpected advantages over the prior art. Thanks to the specific formulation of and amounts of ingredients in the glassy matrix, an oxygen-barrier is created around the coacervated capsules. In so doing, the shelf life of an oxygen sensitive active ingredient can be remarkably increased. A further important advantage is the excellent thermo-stability of the encapsulated ingredient. Without wishing to be bound by theory, it is believed that a wall created around an active ingredient by coacervation to obtain coacervated (micro) capsules protects the active ingredient against the deleterious effects of heat. This permits the particles of the present invention to be incorporated in a food product the preparation of which entails a heat treatment. Further advantages include the free-flowing characteristics of the particles of the invention, as well as the reduced odour perceived when smelling the particles.

### Detailed Description of the Preferred Embodiments

The present invention relates to a particle comprising an active ingredient. Particles, in the context of the present invention, may have any form, for example spherical, round-shaped, rod-like, cubic, disk-like, flat, or film-like having smooth or erratic surfaces. Generally, the shape of the particle of the present invention is determined by the manner of preparation of the glassy matrix. The particle may have a mean diameter, length, thickness or other dimension, depending on its shape, in the range of 5µm to 1 cm, preferably 10µpm to 5mm.

The term "mean" as used, for example, in the expression "mean diameter" refers to the arithmetic mean.

Preferably, the particles of the present invention are essentially dry, meaning that they have a water content of less than 10wt.% by weight of particle, more preferably less than 9wt.%, and most preferably less than 8wt.%. In the case of spray-dried particles, the water content is preferably less than 6wt.%.

Percentages, in the context of the present specification, are percentages by weight based on the total weight of dry matter, unless otherwise indicated.

The particles are typically suitable for oral ingestion.

It has been found that the encapsulated capsules of the present invention are particularly effective at maintaining an oxygen barrier and preventing the unwanted release of undesirable flavours or aromas upon storage.

Thus the particles of the present invention preferably encapsulate an active ingredient which is a bitter tasting and/or oxygen-sensitive ingredient. Preferably it is a nutritional supplement and/or a medicament. Preferably, the active ingredient comprises less than 30wt.% of flavour and/or fragrance compounds, more preferably less than 20wt.% even more preferably less than 10wt.%, and most preferably it is substantially or even entirely free of flavour and/or fragrance compounds.

It has been found that the particles of the present invention are capable of providing an excellent barrier preventing the undesirable migration of oxygen into and flavours out of the particle, even for encapsulated active ingredients which are known to be present problems in this respect. Particularly problematic are so called "fish oils". Fish oil is part of a class of oils know as oils rich in polyunsaturated fatty acids (PUFA's). "Oil rich in PUFA's" is defined herein as an oil comprising at least 5wt.% of PUFA's, preferably, at least 10wt.%, more preferably at least 25wt.%, and most preferably at least 35wt.% PUFA's, based on the total weight of oil.

Thus, the present invention is particularly suited to the encapsulation of such ingredients.

Oils rich in PUFA's are typically obtainable from fish, algae or plant sources. Such oils may also be prepared by different methods such as molecular distillation, a process through which the concentration of selected fatty acids may be increased.

The oil rich in PUFA's is preferably an oil rich in omega-3.

More preferably, the oil rich in PUFAs comprises PUFAs selected from eicosapentaenoic acid (EPA), docosahexaenoic acid (DHA), arachidonic acid (ARA), α-linolenic acid, linoleic acid, and a mixture of at least two thereof. Preferably, the oil rich in PUFAs comprises DHA and EPA, and more preferably consists essentially of DHA and EPA.

The particles of the present invention comprise an active ingredient encapsulated by coacervated capsules. Thus, the process involves coacervation. Preferably, coacervated microcapsules are obtained. Microcapsules are defined herein as capsules having a mean diameter of 0.9 - 2000 µm, preferably 2 - 300 µm.

The term "coacervation", for the purpose of the invention, refers to a process for preparing capsules in a system of generally at least two liquid phases, herein referred to as "a coacervation system".

There is ample literature disclosing coacervation. For instance, US-A-05/0067726 and US-A-03/0193102 A1 disclose the formation of multiple coacervates, such as single-core and multi-core microcapsules having multiple shells obtained by coacervation. These microcapsules encapsulate fish oil. Further documents disclosing coacervation include US 2,800,457, US 5,035,896, US 5,603,952, EP 0 856 355, US 6,475,542, US 6,592,916 and WO 04/022221.

In a step of the coacervation process, one phase is suspended in the form of droplets in another phase. It is also possible to suspend solid particles for encapsulation by coacervation. In this case, the solid particles are suspended in a liquid phase.

The liquid suspending phase is generally an aqueous phase, the droplets of the suspended phase generally being called a hydrophobic, organic or oily phase. In a step of coacervation, hydrocolloids are added to the aqueous phase. This may take place before or after suspending the organic phase in the form of droplets.

Generally, in complex coacervation, hydrocolloids are polyelectrolytes. In simple coacervation, charged or uncharged hydrocolloids may be used, such as gelatine, ethylcellulose, methylcellulose, other cellulosic derivatives, dextran, pullulan, any other uncharged polysaccharides, polyvinylalcohol, and any other charged or uncharged synthetic polymer. Examples include protein matter, such as gelatine, soy proteins, pea proteins, whey proteins, beta-lactoglubulin, albumin, any proteins, chitosan, hydrocolloids or synthetic polymers having an isoelectric point, and carbohydrate matter, such as gum Arabic, carboxymethylcellulose, derivatives of cellulose, alginate, pectinates, deriratives of pectinates and carrageenan.

Depending on the nature of the coacervation, one or more hydrocolloids from the same or different chemical categories (proteins, carbohydrates, and other polyelectrolytes) may be used. For instance, in the present invention, complex coacervation preferably comprises the use of gelatin and gum arabic as the hydrocolloids.

During a phase separation step, hydrocolloids aggregate around the suspended droplets. The aqueous phase itself may divide into two distinct phases; an aqueous, hydrocolloidrich, dense coacervate phase, and an aqueous, hydrocolloid-poor phase. The hydrocolloid rich phase preferably forms a layer around the suspended droplets.

Phase separation step may be brought about in various ways. In complex coacervation, it is achieved by changing the charges on one of at least two different kinds of hydrocolloids present in the aqueous phase. In simple coacervation, phase separation may be achieved by modifying the solubility of a hydrocolloid in another way. In the present invention, simple and complex coacervation are equally suitable to prepare the coacervated capsules.

In general, phase separation may be achieved by modification of properties of the aqueous phase, for example by changing pH, temperature, additives (salts, etc) and/or concentration of its components.

In a particularly preferred embodiment, a hardened wall is formed around the coacervated capsules. Generally, the hardening step may be performed by any method known in the art, and depends on the nature of the coacervation process.

In a preferred method, the wall of the hydrocolloids are cross-linked to obtain a hardened wall around the suspended droplets. In this way, capsules comprising a core corresponding to the suspended droplets and a wall formed by hardened hydrocolloids are obtained. Cross-linking, for the purpose of the present invention, refers to the formation of covalent bonds between hydrocolloids forming the wall.

Thus, the invention also relates to a coacervated capsule comprising a cross-linked wall.

Cross-linking may be performed enzymatically, for example, by the action of transglutaminase. Alternatively, cross-linking may be made with chemical agents, e.g. glutaraldehyde. Enzymatic cross-linking is preferred since the resulting particles are free of glutaraldehyde.

The skilled person is capable of adjusting the droplet size of the suspended phase comprising the active ingredient, and can thus determine the size of the capsules to be obtained following the hardening step. For example, droplet size in a coacervation process may be adjusted with a high speed homogenizer, or by adjusting the stirring speed with a disperser.

Accordingly, in an embodiment of the present invention, the coacervated capsules are microcapsules that have a mean diameter in the range of 0.9 - 300 µm.

In a further embodiment, the coacervated capsules are multiple coacervates, wherein microcapsules form inner coacervated capsules, groups of more than two of which are further encapsulated in a capsule forming an outer coacervated capsule around the group of inner coacervated capsules. Accordingly, the coacervated capsules are preferably at least doubly coacervated capsules, obtainable by forming at least one outer wall by coacervation around coacervated microcapsules. In particles comprising a multiple coacervate, the microcapsules forming the inner coacervated capsules preferably have a mean diameter in the range of 0.9 - 40 µm, preferably 1.1 - 35 µm, most preferably 1.5 - 20 µm and the outer coacervated capsules preferably have a mean diameter in the range of 10 - 300 µm, preferably 50 - 150 µm, more preferably 60 - 100 µm.

Accordingly, the particles of the present invention preferably comprise coacervated capsules having a mean diameter of 0.9 - 150 µm, preferably 1 - 100 µm, more preferably 1.1 - 50µm, and most preferably 1.5-50 µm.

The small size of the coacervated capsules of the present invention provides an important advantage in that, when consumed orally, they are more likely to escape being crunched or ground between the consumer's teeth and thereby to pass undamaged to the gastro-intestinal tract. For this reason, the particles of the present invention preferably comprise primary coacervated capsules having a mean diameter of less than or equal to 150 µm or even more preferably less than or equal to 100 µm. Primary coacervated capsules means capsules forming a capsule alone, in contrast to capsules where several coacervated capsules are further encapsulated by coacervation to provide a secondary or outer coacervate.

Following coacervation, the capsules may be separated from remaining water, the water typically constituting about 60-90wt.% of the coacervation system, including aqueous and hydrophobic phases. Water can be removed in any suitable manner, e.g. decantation and drying.

Alternatively, remaining water may only partially or not at all be removed following coacervation. This has the advantage that a further drying step may be omitted, making the process more economical. The coacervation system may thus be directly used in the step of forming the glassy matrix around the coacervated capsules.

In the particles of the present invention, the coacervated capsules are further encapsulated in a specially formulated glassy matrix.

A glassy matrix, for the purpose of the present invention, is an amorphous solid characterized by viscosities of the order in the range of about 10¹⁰ to 10¹² Pa.s and an extremely low molecular mobility. The presence of a glassy state can be confirmed by establishing characteristic differential scanning calorimetry curves, for which particles are generally brought to the rubbery state by slow and continuous heating of the chamber of the calorimeter to at least the Tg. A good understanding of the glassy state is provided by Dominique Champion et al in "Towards an improved understanding of glass transition and relaxations in foods: molecular mobility in the glass transition range", Trends in Food Science and Technology 11 (2000) 41-55.

For the purpose of the present invention, glass transition temperature (and hence the presence of a glassy state) is determined by calorimetry using a Mettler Toledo DSC822e Differential Scanning Calorimeter (obtainable from Mettler-Toledo GmbH PO Box VI-400, CH-8606 Greifensee Switzerland. Measurement is made of a quantity of 10 mg glassy particles in a 40 microliter sealed aluminium pan. Two scans from -20°C to 95°C at 10°C/ min with a fast cooling at 200°C /min in between are made. The peak of the derivative curve of the second scan is taken as the glass transition value (Tg).

Further explanation on the glassy state is given in: Fan L. T. and Singh S. K., Controlled release: a quantitative treatment, in Polymer Properties and Applications 13, 1989, Springer Verlag; and, D. Benczédi, Flavor encapsulation using polymer-based delivery systems, in Food Flavor Technology, A. J. Taylor ed. 2002, 152-166, Sheffield Press.

Glassy matrices may generally be obtained by cooling a molecular liquid below its glass transition temperature. In the case of polymeric liquids (i.e. thermoplastic melts) a glassy matrix can also be obtained by reducing the level of plasticizer (e.g. water) present in the melt until its glass transition temperature (Tg) exceeds the temperature T to which the carrier is exposed (T-Tg) less than 0. The glassy solidification of a liquid thus occurs whenever a molecular system is cooled or deplasticized at a rate preventing its rearrangement into a well-organised crystalline lattice.

In a preferred embodiment, the glassy matrix used in the present invention has a Tg of above 25°C, more preferably above 30°C, even more preferably above 35°C and most preferably above 40°C. With such a Tg, the particles of the present invention are generally free-flowing and non-sticky, when exposed to ambient conditions.

The glassy matrix around the coacervated capsules may be prepared by spray drying, extrusion, spray granulation, spray coating and/or spray agglomeration together with the matrix components of the glassy matrix.

The processes of spray drying, extrusion, spray granulation, and spray agglomeration are well-known to the skilled person. In the context of the present invention, coacervated particles are mixed with the further components of the glassy matrix and the water content is adjusted to a value typical for the respective process as mentioned above, prior to subject the resulting melt to the respective process.

The components of the glassy matrix, hereinafter referred to as "matrix components" are selected from a narrow range of ingredients and are provided in specific amounts so as to provide the optimal oxygen barrier and flavour retention properties.

A first component of the matrix is a starch hydrolysate.

Suitable starch hydrolysates include maltodextrin and corn syrup. The starch hydrolysate preferably has a mean dextrose equivalence ("DE") of from 5 to 25, more preferably from 6 to 23, even more preferably from 10 to 20 and most preferably from 15 to 19.

The starch hydrolysate is present in an amount of from 50 to 97% by weight based on the total weight of the glassy matrix, more preferably from 60 to 95%, most preferably from 70 to 93%, e.g. from 75 to 90%.

A second component of the matrix is a hydrophobically modified starch and more preferably an alkenyl-succinated starch.

The alkenyl-succinated starch preferably has a degree of substitution of from 0.001 to 0.9. The degree of substitution denotes the number of alkenylsuccinic functional groups per glucose units. Thus, a degree of substitution of 0.001 means that there is 1 alkenylsuccinic functional group per 1000 glucose units. The degree of substitution is more preferably from 0.005 to 0.3 and most preferably from 0.01 to 0.1, e.g. from 0.015 to 0.05.

The alkenyl-succinated starch is preferably a C3 to C14 alkenyl-succinated starch, more preferably C4 to C12, most preferably C5 to C10, e.g. C7 to C9.

Most preferably the alkenyl-succinated starch is octenyl-succinated starch. Ideally the octenyl-succinated starch has a degree of substitution no greater than 0.03, more preferably no greater than 0.02.

The hydrophobically modified starch is present in an amount of from 3 to 50% by weight based on the total weight of the glassy matrix, more preferably from 5 to 40%, most preferably from 7 to 30%, e.g. from 10 to 25%.

In addition to these essential components, other ingredients may be present. Suitable additional ingredients include polymers, such as proteins, polymeric carbohydrates, and other polymeric materials. The polymeric materials preferably comprise hydrophilic polymers in order to improve further the oxygen-barrier properties. Accordingly, the matrix may comprise hydrocolloids. In addition, more hydrophobic polymers may be present in the matrix in order to provide some lipophilic character to the glassy matrix and thus to protect better the particles from moisture. In addition, the matrix may contain further components that are not polymeric, but that may assist in the formation of a dense glassy matrix or that may be added for another purpose. Examples of suitable non-polymeric components include disaccharides.

Suitable proteins include caseins, whey proteins, soy protein, and/or gelatine. These proteins have good emulsification and film-forming properties.

The matrix component may also comprise monosaccharides, such as pentoses or hexoses or mixtures thereof. Suitable pentoses include D-Apiose, L-Arabinose, 2-Deoxy-D-ribose, D-Lyxose, 2-O-Methyl-D-xylose, D-Ribose, D-Xylose. Suitable hexoses include L-Fucose, L-galactose, D-Galactose, D-Glucose, D-Mannose, L-Rhamnose, and L-mannose.

As mentioned above, dissacharides are also possible useful matrix components.

Mono- and dissacharides may be reduced to the corresponding alcohols, e.g. xylitol, sorbitol, D-mannitol and/or maltitol. Similarly, oxidation to aldonic, dicaroxyclic acids or uronic acids and reactions with acids, alkalis or amino compounds can give rise to many other compounds, e.g. isomaltol, which may be comprised in the matrix component.

The matrix component may comprise mixtures of the above- and/or below mentioned carbohydrates, their derivatives and/or proteins. For example, mono-, di- or trisaccharides and/or their reaction products (supra) may be used as additives together with a protein or polysaccharide based matrix and thus bring properties as desired to the matrix component.

The matrix component may comprise oligosaccharides containing from 3-10 monosaccharide units, such as maltopentaose, fructo- and/or galactooligosaccharides.

Alternatively and/or additionally, the matrix component may comprise polysaccharides containing more than 10 monosaccharide units per molecule. These polysaccharides can be linear (cellulose, amylose), and/or branched (amylopectin, glycogen). They can include carboxyl groups (pectin, alginate, carboxymethyl cellulose) or strongly acidic groups (furcellaran, carragenan or modified starch). They can be modified chemically by derivatization with neutral substituents (in the case of methyl ethyl cellulose or hydroxypropyl cellulose for instance) or acidic substituents (with carboxymethyl, sulfate or phosphate groups).

The matrix component may comprise gums and/or hydrocolloids, e.g. gum arabic, gum tragacanth, karaya gum, seaweed or shell extracts like agar, carrageenan, fucoidan, alginic acid, laminaran, furcellaran and/or chitosan, or microbial polysaccharides e.g. dextran, pullulan, elsinan, curdlan, scleroglucan, levan, xanthan, gellan, welan gum and rhamsan gum.

In addition, gum ghatti, gum, karaya gum, laminaran or pectins may be used in the formulation of the matrix component.

Further parameters and components of the process of forming of a glassy matrix around the coacervated capsules depend on the respective process selected. The skilled person is capable through simple trial and error experimentation of adjusting these parameters and/or adding these components.

According to a preferred embodiment, the glassy matrix is formed by screw extrusion. In this case, a mixture may be prepared comprising the matrix components, the coacervated capsules and optional additives, e.g. a lubricating agent. By further addition of water, the viscosity may be adjusted to the required value. For screw-extrusion, typically 5 - 10wt.% and preferably 7 - 9wet. % of total water are present in the mixture prior to feeding the mixture to a screw-extruder, and, extruding the mixture through a die. Total water refers to residual water present in the components of the mixture plus water added to the mixture. At the die, the extruded glassy matrix comprising the coacervated capsules may be chopped into particles. The screw-extruded particles preferably have a water content of 5 - 10wt.%.

In another embodiment of the present invention, the glassy matrix is formed by spray-drying. In this case, a mixture comprising mainly the matrix components, the coacervated capsules and water is prepared. Again, the addition of water helps to adjust the viscosity to the values required for spray drying. The mixture intended for spray-drying may comprise 30 - 99wt.% of water, preferably 35 - 80wt.%, more preferably 40 - 65wt.%. The mixture is then spray dried to remove the water and to produce the particles. The spray dried particles preferably have a water content of 1 - 5wt%.

In still a further embodiment of the present invention, the glassy matrix is made by spray-agglomeration or spray-coating. In this case, a solution comprising the matrix components and water may be prepared. Typically, this solution may comprise 10 - 99wt% of water, preferably 30 - 80wt%, more preferably 40 - 65wt%. The dry coacervated capsules are preferably placed into a fluid-bed chamber where they are fluidised by an upward hot gas stream. The solution of matrix components is then preferably sprayed onto the fluidised dry coacervates and dried simultaneously by the hot gas stream in order to induce agglomeration and/or coating of the coacervates by a dry glassy matrix.

Depending on the size of the coacervated capsules and other factors, agglomeration or simple coating can be performed. More particularly, with coacervates of less than 200µm, agglomeration may hardly be prevented. In contrast, if the coacervated capsules are equal to or greater than 200µm, the skilled person will be able to spray-coat or spray agglomerate by modifying process parameters, such as the water content of the solution or the spraying rate, for example.

In a further embodiment, the glassy matrix is made by spray granulation. In this case, a mixture comprising mainly the matrix components, the coacervated capsules and water is prepared. This mixture may comprise 30 - 99wt% of water, preferably 35 - 80wt%, more preferably 40 - 65wt%. The skilled person may vary process of spray granulation according to preferences. For example, the mixture may be sprayed through a nozzle into an empty chamber of a fluid-bed machine, in which it is dried by the fluidizing hot gas stream. In this case, small particles resulting from the spray drying of the mixture are formed *in situ* and will serve as seeds. These seeds will preferably be repeatedly driven towards the nozzle zone by the fast moving gas turbulences and will be further coated by fresh droplets of the wet mixture exiting the nozzle. Generally, the seeds will then grow and become granules while part of the wet mixture droplets will form new seeds. Such a process may be either performed in batch or in continuous mode. In the latter, granules are continuously discharged from the chamber.

Another example of spray granulation suitable for use in the present invention is given in WO-A2-02/47492 from page 4 line 8 to page 13 line 12.

In yet another embodiment, the glassy matrix is made by extruding a mixture comprising the matrix components, the coacervated capsules, water, and optionally, further additives into a cold solvent. Accordingly, matrix components are preferably dissolved in water and heated to reduce water content to about 6 - 10wt.%. Thereafter, an emulsifier and coacervated capsules may be added to the mixture and uniformly dispersed, for example by agitation or stirring. The mixture may be extruded through holes of about 0.5 - 2 mm under 2.5 - 6 bar into a receiving vessel containing a cold solvent, for example at 2°C or lower, preferably lower than 0°C, even more preferably -3°C or lower. The resulting rods may be reduced in size, e.g. by chopping. The particles may be removed from the cold solvent and dried.

According to a preferred embodiment, the wall around the active ingredient obtained by coacervation forms an inner coating and the glassy matrix forms an outer coating. Further coatings may be applied, if desired.

The active ingredients in coacervated capsules and further encapsulated in a glassy matrix are stabilised in the resulting particles. "Stability" or "stabilising", in the context of the present invention, and more particularly in the context of an oil rich in PUFA, may be determined organoleptically and is defined as having reduced occurrence of typical oxidation products (off notes).

The undesired or unpleasant taste, off note or bitterness of the active ingredient, when present in the particles of the present invention is substantially reduced if compared to active ingredients not encapsulated according to the present invention. The off-notes and unpleasant taste may be assessed by sniffing and the bitterness and/or acidity of the active ingredient in the particles may be assessed through ingesting, chewing and swallowing the particles. In both cases, a comparison can be made with identical active ingredients which are not encapsulated.

The organoleptic acceptability (undesired or unpleasant taste, off note or bitterness, for example) of food products containing encapsulated or un-encapsulated nutrients or other functional food additives is preferably assessed by a panel comprising at least 20 trained panelists. Preferably, the panelists are experienced in assessing and/or evaluating flavours in foods. Differences are preferably analyzed with a Duncan comparison mean at 90% of confidence.

The food product comprising the particles preferably has a water activity of below 0.5, more preferably below 0.45, more preferably below 0.4, more preferably below 0.35, more preferably below 0.3 and most preferably below 0.25, 0.2, 0.15 or even below 0.1. With low availability of free water in a food product, the matrix of the particles remains intact for a longer time and thus better protects the active ingredient from oxygen.

Water activity is preferably measured with an Aqualab CX-2 apparatus (Decagon Devices, Inc., Pullman, Washington, USA) used in accordance with the user's manual.

In an embodiment, a food product for use in invention is selected from an instant soup, a breakfast cereal, a powdered milk, a baby food, a powdered junior drink, a powdered chocolate drink, a spread, a powdered cereal drink, a chewing gum, an effervescent tablet, a cereal bar, and a chocolate bar.

The powdered milks or. drinks are products which are usually consumed after reconstitution of the product with water, milk and/or a juice, or another aqueous liquid.

The food product for use in the invention is preferably a particulate or powdery food. The particles of the invention may easily be added thereto by dry-mixing.

The following examples represent particular embodiments of the present invention.

### Examples

### Examples I and 2

### Preparation of Coacervated Microcapsules by a Complex Coacervation

A stock solution ("A") of gelatine (pork gelatine type A, 275 Bloom) was prepared by mixing 180g of warm deionised water and 20g of gelatine in a vessel until completely dissolved; the solution was then warmed and maintained at 50°C.

A stock solution ("B") of gum Arabic and gum Arabic (Efficacia^{®}, from CNI) was prepared by mixing 180g of cold deionised water and 20g of gum Arabic in a vessel until completely dissolved; the solution was then warmed and kept at 50°C. 24.2g of solution A was mixed with 24.2g of solution B in a vessel under gentle agitation (the gelatine/gum Arabic weight ratio is 1:1). The pH was adjusted to 4.5 with a 50% w/w aqueous lactic solution.

33.9g of fish oil was slowly added to the gelatine and gum Arabic mixture and homogenised with an Ultra-Turrax^{®} disperser at 5000 RPM during 5min, so as to reach an average droplet size of 1 0-20µm. The high shear mixing was stopped and the emulsion was then stirred with a standard stirrer for the remaining of the process in order to avoid coalescence of the droplets. The system was then diluted by the addition of 117.7g of warm deionised water, to bring the total hydrocolloid concentration to 3%w/w. The mixture was then cooled to 10°C at a rate of 0.5°C.min⁻¹ The stirring speed was slightly decreased, and then, according to example 1, the pH was adjusted to 7, 0.97g of transglutaminase (ACTIVE^{®} WM supplied by Ajinomoto) was added to the mixture and cross-linking was allowed to proceed overnight at 10°C whereas, according to example 2, 0.10g of 50%w/w Glutaraldehyde was added to the mixture and cross-linking was allowed to proceed overnight at 20°C.

The experiments were repeated and the obtained slurries of coacervates dried according to any of the following three methods (a), (b) and (c) below. The resulting products are designated 1a, 1b, 1c, 2a, 2b and 2c (1(a) denoting example 1 dried according to method (a) and so on):

### (a) Spray drying to obtain a glassy matrix

69.5g of a mixture of 90% maltodextrine 18DE (ex. Roquette) and 10% modifies starch (Capsul^{®}, ex. National Starch) was added to the coacervate suspension and thoroughly mixed, at room temperature, until completely dissolved. The coacervate suspension was then spray dried on a pilot plant spray drier (Niro FSD0.8) equipped with a rotating wheel operated at 30000RPM. The inlet temperature was 200°C, whilst the outlet temperature was kept at 85°C by controlling the pump speed. The result was a free-flowing powder of particles comprising coacervated microcapsules having a mean diameter of 50µm, the microcapsules being further encapsulated in a glassy matrix comprising polymeric carbohydrate material.

### (b) Spray drying in the absence of a glassy matrix

1g of anti-caking agent (silicone dioxide) was added to the slurry of coacervates as processing aid. The coacervate suspension was then spray dried on a pilot plant spray drier (Niro FSD0.8) equipped with a rotating wheel operated at 30000RPM. The inlet temperature was 150°C, while the outlet temperature was kept at 100°C by controlling the pump speed. A free-flowing powder was produced.

### (c) Spray Granulation to obtain a glassy matrix

69.5g of a mixture of 90% maltodextrine 18DE (ex. Roquette) and 10% modified starch (Capsul^{®}, ex. National Starch) was added to the coacervate suspension and thoroughly mixed, at room temperature, until completely dissolved. This suspension was sprayed into a pilot plant fluidised bed (Glatt AGT150) equipped with a 2mm two-fluid nozzle mounted for bottom spray and operating at 1 bar atomising gas pressure. The inlet temperature was 120°C, while the outlet temperature was kept at 60°C by controlling the speed of the peristaltic pump. The fluidising/drying gas flow was set to 140 m³/h. The fluidised bed dryer was operated in continuous mode using a zig-zag air classifier (gas flow set at 0.2 bar) to continuously discharge the granules and control the average desired particle size. The result was free-flowing spherical granules in which the coacervated microcapsules are embedded in a glassy carbohydrate matrix. The average particle size was 400 microns.

### Examples 3 and 4

### Glassy Matrix Encapsulating Coacervated Microcapsules by Screw-Extrusion

In examples 3a, 3b 3c and 4a, coacervated capsules were encapsulated by screw extrusion. In 3a, dried coacervated capsules of 1 b were used, for 3b those of 2b were used, for 3c doubly coacervated particles as disclosed in WO 04/041251 were used (doubly coacervated particles of this type are commercially available as MEG-3^{™} from Ocean Nutrition, Canada (ONC). For 4a, coacervated capsules of 1a are used.

In 3a, 3b and 3c, prior to extrusion, a blend was prepared by mixing, in a high shear mixer, 200g of dry coacervated capsules, 252g of maltodextrine 18DE (ex. Roquette Frères), 28g of modified starch (Capsul^{®} ex. National Starch) and 5g of lubricating agent (internal number 53128). During addition of these components, 15g of water was also added slowly to the mixer to adjust the viscosity of the final product.

In 4a, prior to extrusion, a blend was prepared by mixing, in a high shear mixer, 475g of dry coacervated capsules and 5g of lubricating agent (internal number 53128). During addition of these components, 20g of water was also added slowly to the mixer to adjust the viscosity of the final product.

For each example, the mixture was then fed to a ThermoPRISM twin screw co-rotative extruder model KX-16Eurolab. The extruder has a low shear screw configuration, a barrel diameter of 16mm, one die opening of 2mm diameter and a L/D of 25:1. The screw speed was set to 200RPM at a rate of 0.6 kg.h⁻¹. By adjusting the barrel temperature profile at 110°C-100°C-20°C-20°C the die pressure was maintained between 1 and 5 bar.

The melt product leaving the die of the extruder was chopped into discrete spherical beads with a chopper at 60RPM. The granules were then conveyed and cooled in a cyclone at room temperature.

### Examples 5 and 6

### Glassy Matrix Encapsulating Coacervated Microcapsules by Spray Drying or Spray Granulation

Dried coacervated capsules of examples 1a, 1b and 3c were surrounded by a glassy matrix by spray-drying to provide examples 5a, 5b, 5c or spray-granulation to provide examples 6a, 6b, and 6c.

A solution was prepared by mixing 225g of maltodextrine 18DE (supply by Roquette Frères), 25g of modified starch (Capsul^{®} from National Starch) and 700g of cold water in a vessel until it was completely dissolved; the solution was then maintained under gentle agitation. 250g of dried coacervated capsules were added and thorough mixing took place at room temperature until a completely homogeneous coacervate suspension formed.

In example 5a, 5b, and 5c, the coacervate suspension was spray dried on a pilot plant spray drier (Niro FSD0.8) equipped with a rotating wheel operated at 30000RPM. The inlet temperature was 210°C, while the outlet temperature was kept at 85°C by controlling the speed of the peristaltic pump. The result was a free-flowing powder.

In examples 6a, 6b, and 6c, the coacervate suspension was sprayed into a pilot plant fluidised bed (Glatt AGT150) equipped with a 2mm two-fluid nozzle mounted for bottom spray and operating at 1 bar atomising gas pressure. The inlet temperature was 120°C, while the outlet temperature was kept at 60°C by controlling the speed of the peristaltic pump. The fluidising/drying gas flow was set to 140 m³/h. The fluidised bed dryer was operated in continuous mode using a zig-zag air classifier (gas flow set at 0.2 bar) to continuously discharge the granules and control the average desired particle size. The result was free-flowing granules having an average particle size of 400 microns.

### Example 7

### Glassy Matrix Encapsulating Coacervated Microcapsules by Spray Agglomeration.

Dried coacervated capsules of examples 1a, 1b and 3c were surrounded by a glassy matrix by spray-agglomeration to provide sample 7a, 7b and 7c.

A solution ("C") was prepared by mixing 225g of maltodextrine 18DE (ex. Roquette Frères), 25g of modified starch (Capsul^{®} ex. National Starch) and 250 g of cold water in a vessel until it was completely dissolved; the solution was then maintained under gentle agitation at 50°C.

An Acromatic Fielder Streal fluidized bed was fed with 250g of dried coacervated capsules and solution C was then sprayed into the fluidised bed equipped with a 1mm two-fluid nozzle mounted for bottom spray and operating at 2 bar atomising gas pressure. The inlet temperature was 80°C, while the outlet temperature was kept at 60°C by controlling the speed of the peristaltic pump. The result was a free-flowing agglomerate having an average particle size of about 200 microns.

**Table 1 : Characteristics of the Particles**

| Example | Size of coacervated capsules | Mean particle size | Fish Oil load | Water content |
|---|---|---|---|---|
| 1A | 20µm | 50µm | 30% | about 4% |
| 1B | 20µm | 20µm | 80% | about 2% |
| 1C | 20µm | 400µm | 30% | about 4% |
| 2A | 20µm | 50µm | 30% | about 4% |
| 2B | 20µm | 20µm | 80% | about 2% |
| 3A | 20µm | 2200µm | 26% | about 8% |
| 3B | 20µm | 2200µm | 26% | about 8% |
| 3C | 40µm | 2200µm | 21% | about 8% |
| 4 | 50µm | 2200µm | 27% | about 8% |
| 5A | 20µm | 50µm | 35% | about 4% |
| 5B | 20µm | 50µm | 35% | about 4% |
| 5C | 40µm | 50µm | 30% | about 4% |
| 6A | 20µm | 400µm | 35% | about 4% |
| 6B | 20µm | 400µm | 35% | about 4% |
| 6C | 40µm | 400µm | 30% | about 4% |
| 7A | 20µm | 200µm | 35% | about 4% |
| 7B | 20µm | 200µm | 35% | about 4% |
| 7C | 40µm | 200µm | 30% | about 4% |

## Claims

1. A particle comprising an active ingredient which is encapsulated in one or more coacervated capsules, the one or more coacervated capsules being further encapsulated in a glassy matrix, wherein the glassy matrix comprises:
(i) 3 - 50wt% of a hydrophobically modified starch, and
(ii) 50 - 97wt% of a starch hydrolysate.

2. A particle according to claim 1 wherein the hydrophobically modified starch is an alkenyl-succinated starch.

3. A particle according to claim 2 wherein the hydrophobically modified starch is a C3 to C 14 alkenyl-succinated starch.

4. A particle according to claim 3 wherein the alkenyl-succinated starch is octenyl-succinated starch.

5. A particle according to any one of the preceding claims wherein the starch hydrolysate has a dextrose equivalence of from 5 to 25.

6. A particle according to any one of the preceding claims, in which the active ingredient is an oil rich in polyunsaturated fatty acids (PUFAs).

7. A method of preparing a particle encapsulating an active ingredient, the method comprising the steps of encapsulating an active ingredient by coacervation to obtain one or more coacervated capsules, and forming a glassy matrix around the one or more coacervated capsules, wherein the glassy matrix comprises, as matrix components:
(i) 3 - 50wt% of a hydrophobically modified starch, and
(ii) 50 - 97wt% of a starch hydrolysate.

8. A method according to claim 7, wherein the step of forming a glassy matrix around the coacervated capsules is made by spray drying, spray granulating, spray agglomerating and/or extrusion of the coacervated capsules together with the matrix components.

9. A method according to either claim 7 or claim 8, in which the process of coacervation comprises the step of cross-linking one or more hydrocolloids that form a layer around the active ingredient.

10. Use of a glassy matrix as defined in any one of claims 1 to 5 encapsulating one or more coacervated capsules to mask undesired and/or unpleasant taste, off-note or bitterness of an active ingredient intended for oral ingestion.

## Patentansprüche

1. Teilchen, umfassend einen Wirkstoff, welcher in eine oder mehrere koazervatierte Kapseln eingekapselt ist, wobei die eine oder mehreren koazervatierten Kapseln weiter in eine glasartige Matrix eingekapselt sind, wobei die glasartige Matrix umfasst:
(i) 3-50 Gew.-% einer hydrophob modifizierten Stärke, und
(ii) 50-97 Gew.-% eines Stärkehydrolysats.

2. Teilchen nach Anspruch 1, wobei die hydrophob modifizierte Stärke eine Alkenyl-succinierte Stärke ist.

3. Teilchen nach Anspruch 2, wobei die hydrophob modifizierte Stärke eine C₃- bis C₁₄-Alkenyl-succinierte Stärke ist.

4. Teilchen nach Anspruch 3, wobei die Alkenyl-succinierte Stärke Octenylsuccinierte Stärke ist.

5. Teilchen nach einem der vorhergehenden Ansprüche, wobei das Stärkehydrolysat eine Dextroseäquivalenz von 5 bis 25 hat.

6. Teilchen nach einem der vorhergehenden Ansprüche, in welchem der Wirkstoff ein Öl, reich an mehrfach ungesättigten Fettsäuren (PUFAs), ist.

7. Verfahren zum Herstellen eines Teilchens, einkapselnd einen Wirkstoff, wobei das Verfahren die Schritte umfasst, einen Wirkstoff durch Koazervation einzukapseln, um eine oder mehrere koazervatierte Kapseln zu erhalten, und eine glasartige Matrix um die eine oder mehreren koazervatierten Kapseln herum zu erzeugen, wobei die glasartige Matrix als Matrixkomponenten umfasst:
(i) 3-50 Gew.-% einer hydrophob modifizierten Stärke, und
(ii) 50-97 Gew.-% eines Stärkehydrolysats.

8. Verfahren nach Anspruch 7, wobei der Schritt, eine glasartige Matrix um die koazervatierten Kapseln herum zu erzeugen, durch Sprühtrocknen, Sprühgranulieren, Sprühagglomerieren und/oder Extrusion der koazervatierten Kapseln zusammen mit den Matrixkomponenten durchgeführt wird.

9. Verfahren nach entweder Anspruch 7 oder Anspruch 8, in welchem das Verfahren der Koazervation den Schritt umfasst, ein oder mehrere Hydrokolloide zu vernetzen, die eine Schicht um den Wirkstoff herum erzeugen.

10. Verwendung einer glasartigen Matrix, wie definiert in einem der Ansprüche 1 bis 5, einkapselnd eine oder mehrere koazervatierte Kapseln, um unerwünschten und/oder unangenehmen Geschmack, Beigeschmack oder Bitterkeit eines Wirkstoffs, vorgesehen für orale Einnahme, zu maskieren.

## Revendications

1. Particule comprenant un ingrédient actif qui est encapsulé dans une ou plusieurs capsules coacervées, la ou les capsules coacervées étant en outre encapsulées dans une matrice vitreuse, ladite matrice vitreuse comprenant:
(i) 3 à 50% en poids d'un amidon modifié hydrophobiquement, et
(ii) 50 à 97% en poids d'un hydrolysat d'amidon.

2. Particule selon la revendication 1 dans laquelle l'amidon modifié hydrophobiquement est un alcénylsuccinate d'amidon.

3. Particule selon la revendication 2 dans laquelle l'amidon modifié hydrophobiquement est un (alcényle en C₃-C₁₄)succinate d'amidon.

4. Particule selon la revendication 3 dans laquelle l'alcénylsuccinate d'amidon est un octénylsuccinate d'amidon.

5. Particule selon l'une quelconque des revendications précédentes dans laquelle l'hydrolysat d'amidon a un dextrose équivalent de 5 à 25.

6. Particule selon l'une quelconque des revendications précédentes, dans laquelle l'ingrédient actif est une huile riche en acides gras polyinsaturés (AGPI).

7. Procédé de préparation d'une particule encapsulant un ingrédient actif, le procédé comprenant les étapes consistant à encapsuler un ingrédient actif par coacervation pour obtenir une ou plusieurs capsules coacervées, et à former une matrice vitreuse autour de la ou des capsules coacervées, la matrice vitreuse comprenant, en tant que constituants de matrice:
(i) 3 à 50% en poids d'un amidon modifié hydrophobiquement, et
(ii) 50 à 97% en poids d'un hydrolysat d'amidon.

8. Procédé selon la revendication 7, dans lequel l'étape de formation d'une matrice vitreuse autour des capsules coacervées se fait par séchage par atomisation, granulation par atomisation, agglomération par atomisation, et/ou extrusion des capsules coacervées conjointement avec les constituants de matrice.

9. Procédé selon la revendication 7 ou la revendication 8, dans lequel l'opération de coacervation comprend l'étape consistant à réticuler un ou plusieurs hydrocolloïdes qui forment une couche autour de l'ingrédient actif.

10. Utilisation d'une matrice vitreuse telle que définie dans l'une quelconque des revendications 1 à 5 encapsulant une ou plusieurs capsules coacervées pour masquer un goût indésirable et/ou désagréable, une fausse note gustative ou une amertume d'un ingrédient actif destiné à une ingestion orale.
